(19) Europäisches Patentamt
European Patent Office
Office européen des brevets

(11) **EP 3 071 145 B1**

(12) **FASCICULE DE BREVET EUROPEEN**

(45) Date de publication et mention de la délivrance du brevet:
**11.10.2017 Bulletin 2017/41**

(21) Numéro de dépôt: **14798743.2**

(22) Date de dépôt: **04.11.2014**

(51) Int Cl.:
**A61F 2/06** *(2013.01)*        **A61F 2/07** *(2013.01)*
**A61F 2/954** *(2013.01)*

(86) Numéro de dépôt international:
**PCT/EP2014/073735**

(87) Numéro de publication internationale:
**WO 2015/071135 (21.05.2015 Gazette 2015/20)**

(54) **PROTHÈSE ENDOVASCULAIRE POUR MONTAGE EN CHEMINÉE**

KAMINIMPLANTAT-STENT

CHIMNEY-GRAFT STENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **18.11.2013 FR 1361292**

(43) Date de publication de la demande:
**28.09.2016 Bulletin 2016/39**

(73) Titulaire: **Mialhe, Claude**
**83300 Draguignan (FR)**

(72) Inventeur: **Mialhe, Claude**
**83300 Draguignan (FR)**

(74) Mandataire: **Decobert, Jean-Pascal**
**Cabinet Hautier**
**Office Méditerranéen de Brevets d'Invention**
**20, rue de la Liberté**
**06000 Nice (FR)**

(56) Documents cités:
**WO-A1-2011/158045      US-A1- 2008 109 066**
**US-A1- 2011 270 380      US-A1- 2012 109 279**
**US-A1- 2013 103 134      US-A1- 2013 289 701**

## Description

DOMAINE TECHNIQUE

La présente invention concerne une prothèse endovasculaire particulièrement pour montage en cheminée dans le corps humain ou animal.

L'invention trouvera son application pour le traitement de pathologies vasculaires notamment le traitement des anévrismes. Plus particulièrement la prothèse selon l'invention est une endoprothèse destinée à être placée en parallèle avec au moins une autre endoprothèse dans un vaisseau sanguin.

ETAT DE LA TECHNIQUE

Un anévrisme est une dilatation localisée de la paroi d'un vaisseau sanguin, notamment d'une artère, aboutissant à la formation d'une poche de taille variable. La rupture d'anévrisme constitue un accident vasculaire pouvant être mortel. Il est donc essentiel de traiter l'anévrisme avant la rupture du vaisseau.

Le traitement d'un anévrisme peut se faire par chirurgie ouverte et, de manière de plus en plus courante, par chirurgie endovasculaire. Cette technique est souvent préférée car moins invasive. Elle consiste à placer à l'intérieur de la zone lésée de l'aorte par exemple, une prothèse pour exclure l'anévrisme de la circulation sanguine. L'endoprothèse vasculaire est mise en place sans ouverture de l'aorte, elle est introduite par d'autres vaisseaux sanguins périphériques.

L'anévrisme peut être localisé dans une zone de l'artère où se situent également des branchements à des vaisseaux secondaires. Il faut alors simultanément exclure l'anévrisme de la circulation sanguine tout en maintenant la circulation sanguine dans les vaisseaux secondaires.

La technique du montage en cheminée consiste à placer une endoprothèse principale dans l'artère à traiter et à insérer des endoprothèses collatérales en parallèle à l'endoprothèse principale pour faire la connexion entre l'artère et les vaisseaux secondaires dont on souhaite maintenir la circulation sanguine. Les endoprothèses collatérales sont agencées en parallèle et à l'extérieure de la prothèse principale. Cette technique permet de garantir la perméabilité de la circulation sanguine dans l'artère au travers d'une endoprothèse principale ainsi que la circulation secondaire tout en excluant le segment anévrismal.

Les avantages principaux de ce montage en cheminée sont la simplicité de mise en place et la disponibilité du matériel par rapport aux autres techniques notamment de montage par fenêtre nécessitant une fabrication sur mesure, et une mise en place d'une extrême précision.

Toutefois, l'inconvénient principal est le risque de fuite entre les deux endoprothèses du fait des conflits mécaniques entre elles pouvant conduire à une endofuite dans le sac anévrismal.

On connait de US-A1-20130103134 une endoprothèse collatérale pour les vaisseaux secondaires présentant une section non circulaire pouvant être déformable pour se conformer, d'un côté à l'endoprothèse principale, et de l'autre à l'artère, de sorte à limiter les endofuites entre les endoprothèses parallèles. Cette technique ne donne pas satisfaction car la déformation de l'endoprothèse collatérale est peu contrôlable et peu stable. La circulation sanguine dans le vaisseau secondaire n'est pas suffisante.

Il existe donc le besoin d'une endoprothèse qui permet à la fois de traiter l'anévrisme tout en améliorant les connexions avec les vaisseaux secondaires.

RESUME DE L'INVENTION

A cet effet, la présente invention propose une endoprothèse vasculaire comprenant dans une de ses portions d'extrémité, préférentiellement en portion proximale, au moins un godet orienté vers ladite extrémité, préférentiellement proximale, de l'endoprothèse formant une dépression dans le pourtour de l'endoprothèse apte à coopérer avec une endoprothèse collatérale.

La présence du godet en portion proximale permet de ménager, dans l'endoprothèse principale, un volume pour l'endoprothèse collatérale tout en optimisant la coopération entre les deux endoprothèses. L'endoprothèse collatérale ne déforme pas, ou dans une moindre mesure, la prothèse principale qui conserve une section sensiblement circulaire tandis que la prothèse collatérale est elle aussi orientée dans le prolongement de la connexion avec le vaisseau secondaire sans déformation ou une moindre déformation.

A titre préféré, la prothèse comprend une pluralité de godets sur le pourtour de la prothèse de sorte à faciliter la mise en place de la prothèse sans nécessiter d'ajustement précis, notamment angulaire, pour le passage de la prothèse collatérale.

D'autres buts et avantages apparaitront au cours de la description qui suit d'un mode de réalisation préféré.

Il convient de rappeler que la présente invention concerne une prothèse endovasculaire adaptée au montage en cheminée comprenant une gaine et une armature tubulaires définissant une lumière caractérisée par le fait que la prothèse comprend à une extrémité de la gaine au moins un godet formé en direction de ladite extrémité de sorte à former une dépression dans la lumière, dépression destinée à coopérer avec une prothèse endovasculaire collatérale.

Suivant des variantes préférées mais non limitative de l'invention, la prothèse est telle que :

- le godet est formé par la gaine ;
- au niveau du godet, l'armature est configurée pour laisser le godet libre de déformation ;
- la gaine recouvre au moins partiellement l'armature ;

- la gaine et l'armature sont superposées ;
- la gaine est plus courte que l'armature ;
- l'armature comprend à l'extrémité proximale de la gaine un fil métallique périphérique disposé en « z » et comprenant des sommets proximaux dirigés vers l'extrémité proximale, le au moins un godet étant formé entre deux sommets proximaux successifs ;
- le godet est maintenu par défaut dans une position pliée sous la forme d'un pli d'aisance ;
- le pli d'aisance est maintenu par des moyens de fixation amovible ;
- les moyens de fixation sont agencés sur la face externe de la gaine ;
- le pli d'aisance formé comprend une composante principale orientée suivant l'axe longitudinal de la prothèse ;
- le pli d'aisance aligne le godet à la périphérie de l'armature ;
- la prothèse comprend une pluralité de godets sur le pourtour de la gaine ;
- les godets sont formés entre des sommets proximaux du fils métallique périphérique de l'armature disposé en « z ».

Suivant un autre aspect, l'invention concerne un système de prothèses endovasculaires adapté au montage en cheminée comprenant une prothèse principale et une prothèse collatérale destinées à être assemblées en parallèle dans un vaisseau sanguin humain ou animal caractérisé par le fait que la prothèse principale comprend à une extrémité au moins un godet formé en direction de ladite extrémité de sorte à former une dépression recevant la prothèse collatérale sur une portion de la surface extérieure de la prothèse principale.

Selon un autre aspect séparable de l'invention, le godet a une profondeur maximale égale au diamètre de la prothèse collatérale, préférentiellement la moitié du diamètre.

Avantageusement, la prothèse endovasculaire adaptée au montage en cheminée comprend une armature tubulaire associée à une gaine tubulaire, la prothèse étant configurée pour prendre une position comprimée et une position déployée caractérisée par le fait que la gaine et l'armature sont en contact à une extrémité de la gaine respectivement au niveau au moins de deux points de contact sur la gaine et au moins de deux points d'appui sur l'armature en position déployée la longueur de la gaine entre les deux points de contact de ladite gaine étant supérieure à la distance entre les deux points d'appui de l'armature de sorte à former une dépression dans la prothèse apte à recevoir une prothèse collatérale. Un autre aspect qui ne forme pas de la présente invention concerne un procédé de mise en place d'une prothèse telle que décrite ci-dessus et préférentiellement d'au moins une prothèse collatérale. Le procédé comprend les étapes successives suivantes :

- introduction de la prothèse principale sous forme comprimée dans l'aorte à traiter ;
- déploiement de la prothèse principale,
- introduction d'une prothèse collatérale dans un vaisseau secondaire débouchant au niveau de la prothèse principale ;
- positionnement de la prothèse collatérale partiellement dans le vaisseau secondaire et partiellement en parallèle de la prothèse principale dans l'artère au niveau d'un godet de la prothèse principale ;
- déploiement de la prothèse collatérale.

Avantageusement, la prothèse collatérale est accolée à la surface extérieure de la prothèse principale.

Avantageusement, le déploiement de la prothèse collatérale entraîne le déploiement du pli d'aisance.

Avantageusement, les moyens de fixation du pli d'aisance sont rendus inactifs par le déploiement de la prothèse collatérale.

BREVE DESCRIPTION DES FIGURES

Les figures sont données à titre d'exemple pour illustrer et mieux comprendre l'invention sans être aucunement limitatives.

Figures 1 et 2 : vues en coupe longitudinale de la portion proximale de la prothèse comportant une pluralité de godet en position pliée (figure 1) et en position déployée (figure 2).

Figures 3 à 6 vues en coupe transversale de la prothèse, illustrant plusieurs modes de réalisation de positionnement des godets représentés en position pliée sauf en figure 6 dans laquelle un godet est en position déployée.

Figures 7 à 9 : schémas pour la réalisation du godet selon l'invention.

Figure 10: schéma de principe du positionnement d'un montage en cheminée avec la prothèse selon l'invention placée dans une artère rénale avec une vue en coupe transversale de la coopération des deux prothèses parallèles.

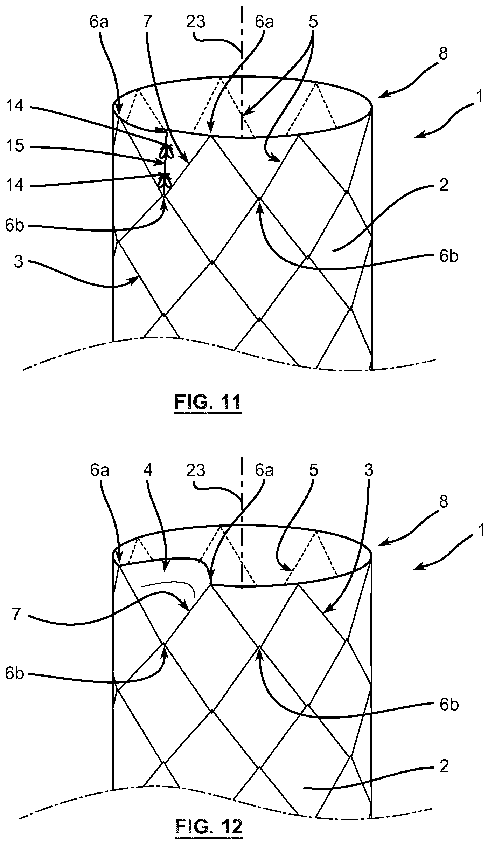

Figures 11 et 12 : vues en perspectives d'une prothèse selon l'invention comprenant un godet en position pliée (Figure 11) sous forme d'un pli d'aisance maintenu par deux moyens de fixation amovible et en position dépliée (Figure 12).

Figure 13 : vue en perspective d'une prothèse de l'état de l'art en position dans une artère avec une prothèse collatérale montée en parallèle.

Figure 14 : vue en perspective d'une prothèse selon l'invention en position dans une artère avec une prothèse collatérale montée en parallèle.

DESCRIPTION DETAILLEE DE L'INVENTION

La présente invention concerne une prothèse endovasculaire également appelée endoprothèse.

Le terme proximal et distal s'interprète par rapport au coeur, proximal étant plus proche du coeur et distal étant plus éloigné.

Le terme prothèse réfère à un dispositif pour l'insertion ou l'implantation dans une partie d'un corps humain ou animal. La prothèse endovasculaire selon l'invention est destinée à être insérée ou implantée dans le système vasculaire d'un corps humain ou animal. Une prothèse endovasculaire est destinée à maintenir la paroi du vaisseau et assurer autant que possible l'intégrité du diamètre de sa lumière. La prothèse est avantageusement biocompatible, elle peut être permanente ou biorésorbable.

La prothèse 1 comprend une extrémité proximale 8, une extrémité distale 9 (toutes deux définissant une embouchure) et une lumière 19 s'étendant le long de la prothèse 1 pour permettre le passage du sang d'une extrémité à l'autre, dans le cas illustré d'une artère de l'extrémité proximale 8 à l'extrémité distale 9.

La prothèse 1 comprend une gaine 2 également appelée tissu prothétique. La gaine 2 est une nappe de matériau. Cette gaine 2 est fabriquée en matériau étanche de sorte à rétablir la circulation sanguine au travers de la lumière 19 de la prothèse 1. La gaine 2 a une forme tubulaire. A titre d'exemple, la gaine 2 est en Gore-tex® ou en Dacron®

La prothèse 1 comprend préférentiellement une armature 3 pour maintenir la gaine 2 en forme. L'armature 3 peut être interne et/ou externe à la gaine 2 ou bien interne sur une partie et externe sur une autre. L'armature 3 est selon une première possibilité entièrement associée à la gaine 2 ou selon une deuxième possibilité, l'armature 3 présente une partie qui n'est pas associée à la gaine 2 ; l'armature 3 est partiellement associée à la gaine 2, notamment en étant plus longue que la gaine. Par exemple, la portion proximale de l'armature 3 est libre de la gaine 2, c'est-à-dire qu'elle n'est pas en contact avec une partie de la gaine, pour faciliter la fixation de la prothèse 1 dans le vaisseau sanguin ; dans ce cas, la gaine 2 est plus courte que l'armature 3. L'armature 3 permet de donner une forme à la prothèse 1 et d'assurer une étanchéité satisfaisante avec le vaisseau dans lequel la prothèse 1 est placée. L'armature 3 permet d'appliquer la prothèse 1 contre les parois du vaisseau.

L'armature 3 présente de préférence une forme tubulaire. Dans la présente invention, le terme tubulaire signifie que la structure considérée définit un chenal central débouchant à deux extrémités par des ouvertures. Géométriquement, la forme tubulaire peut être de toute forme allongée, notamment cylindrique de section circulaire ou non. La forme tubulaire peut éventuellement comprendre une courbure suivant sa direction longitudinale, orientée suivant la longueur du vaisseau à équiper.

L'armature 3 est ajourée, préférentiellement, l'armature 3 n'interfère pas avec des zones de branchement de vaisseaux secondaires. L'armature 3 forme avantageusement un maillage. Un motif de maillage classiquement utilisé est par exemple un fil, stent en anglais, en Z. Pour la suite de la description le terme stent sera utilisé. L'armature 3 comprend avantageusement au moins un stent circulaire formant un zigzag ou Z. Le stent en zigzag comprend une série de segments droits ou traverses 7 interconnectés par une série de segments courbés ou coudes. Les segments courbés peuvent comprendre des angles aigus ou sommets 6. Dans la forme en Z, les segments droits 7 sont agencés en angles, c'est-à-dire non parallèles les uns aux autres et reliés par des sommets 6. L'armature 3 est classiquement métallique, par exemple en matériau tel que le Nitinol® ou en chrome-cobalt, elle peut aussi être en matériau biocompatible et biodégradable par exemple en polymère d'acide lactique.

La prothèse 1, et de ce fait l'armature 3 et la gaine 2, est flexible pour s'adapter à la morphologie des vaisseaux sanguins.

La prothèse 1 est configurée pour être comprimée lors de sa mise en place puis être expansée pour jouer son rôle de maintien du vaisseau et de la circulation sanguine. La prothèse 1 peut être ballon-expansive, c'est-à-dire qu'un ballon est gonflé dans la lumière 19 de la prothèse 1 pour déployer l'armature 3 qui reste ainsi en place une fois le ballon retiré et/ou auto-expansive, c'est-à-dire que la prothèse 1 se déploie seule, de par sa construction dès qu'une contrainte de compression est levée, par exemple par retrait d'un tube d'introducteur dans lequel la prothèse est initialement logée.

La prothèse 1 selon l'invention peut être utilisée placée seule dans le vaisseau principal ou en association avec d'autres types de prothèses connues sur le marché.

La prothèse 1 selon l'invention est destinée à être positionnée dans des vaisseaux sanguins présentant un anévrisme et des branchements à des vaisseaux sanguins secondaires. Typiquement, la prothèse 1 est destinée à être placée au niveau de l'artère rénale ou de la crosse aortique abdominale.

La prothèse 1 selon l'invention est destinée à être placée dans le vaisseau principal, préférentiellement porteur de la pathologie à traiter. Au moins une autre prothèse 10 est placée dans le vaisseau secondaire. Cette prothèse 10 est également une prothèse endovasculaire aussi appelée endoprothèse. Pour la présente description, cette prothèse 10 est dénommée prothèse collatérale 10. La prothèse collatérale 10 présente une structure similaire à la prothèse 1 telle que décrite ci-dessus avec une gaine 2 et une armature 3 hormis le godet 4 décrit ci-après.

La prothèse 1 selon l'invention comprend, de manière caractéristique, en partie, préférentiellement proximale, un godet 4 en direction de ladite extrémité, préférentiellement proximale 8, de la prothèse 1 formant une dépression dans le pourtour de la prothèse 1. Le godet 4 est destiné à coopérer avec une prothèse collatérale 10.

Le godet 4 s'entend comme une ondulation qui va en s'évasant. Le godet 4 est avantageusement formé dans la nappe de la gaine 2. La gaine 2 comprend dans sa partie proximale une ondulation vers la lumière 19 de la prothèse 1 avec un évasement en direction de l'extrémité proximale 8.

Le godet 4 est formé par une surlongueur du périmètre de la gaine 2. Selon un mode de réalisation, la surlongueur est effective sur une hauteur 16 prédéfinie de la gaine 2. La surlongueur peut être fixe sur toute la hauteur 16, formant alors un manchon. Préférentiellement, la surlongueur est croissante, linéairement ou non, sur cette hauteur 16 en direction de l'extrémité proximale de la gaine 2. Il y a formation d'un évasement local. Le profil d'évasement du godet 4 peut être varié. L'accroissement peut être continu ou par palier ou bien encore être nul au moins sur une partie de la hauteur 16.

La hauteur 16 est choisie en fonction du degré de verticalisation voulue pour la prothèse collatérale 10 ainsi que sur l'étendue en hauteur nécessaire en fonction du nombre de vaisseaux secondaires à préserver.

Le godet 4 est avantageusement libre de déformation. L'armature 3 est configurée pour s'adapter au godet 4. Par exemple, l'armature 3 suit la forme du godet 4 dans la lumière 19 de la prothèse. Préférentiellement, l'armature 3, n'interfère pas avec le godet 4. Ce dernier peut donc être dans une zone de la prothèse où l'armature 3 n'est pas présente.

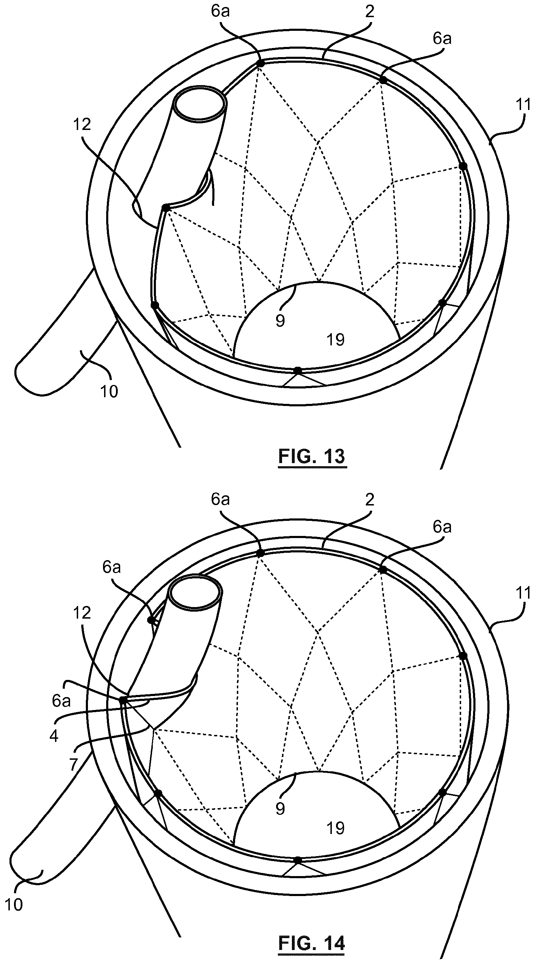

Avantageusement, la prothèse 1 est appliquée sur la paroi interne du vaisseau 10 à la base du godet 4 limitant les risques de fuite du vers le sac anévrismal. Préférentiellement, la prothèse 1 s'applique majoritairement contre la paroi interne du vaisseau de part et d'autre du godet 4 comme illustré en figure 14. Selon une possibilité l'armature 3 contribue à ce contact entre la prothèse 1 au pourtour du godet 4 et la paroi interne du vaisseau 10.

Selon une possibilité préférée, la prothèse 1 comprend au niveau de l'extrémité proximale de la gaine 2 un stent en zigzag. Le godet 4 est avantageusement formé entre deux sommets proximaux 6a successifs. Le godet 4 s'étend alors depuis un sommet distal 6b, entre deux traverses 7 successives et jusqu'au maximum à deux sommets proximaux 6a. Il peut ainsi disposer d'un contour en « V » suivant l'armature 3.

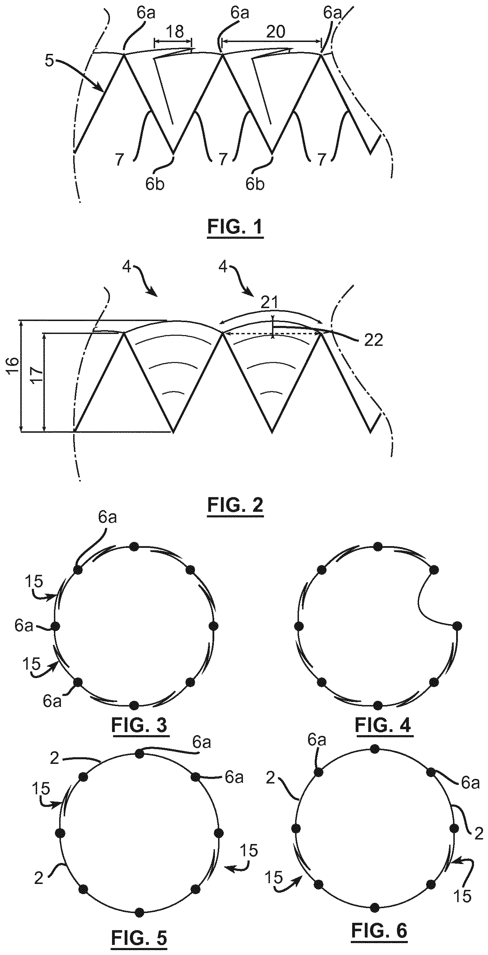

Selon un mode de réalisation avantageux, la prothèse 1 comprend une pluralité de godets 4, avantageusement répartis, sur le pourtour de la gaine 2. Différentes configurations sont représentées aux figures 3 à 6. Préférentiellement, un godet 4 est formé entre chaque sommet proximal 6a du stent en Z à l'extrémité proximale de la gaine 2. La présence de plusieurs godets 4 permet de monter plusieurs prothèses en parallèle et donc maintenir la circulation sanguine dans plusieurs vaisseaux sanguins secondaires 12.

La présence de godets 4 sur tout le pourtour de la gaine 2 ou sur plusieurs secteurs angulaires est ainsi particulièrement intéressante car cette disposition permet de placer facilement la prothèse 1 et au moins une prothèse collatérale 10 sans nécessiter d'orienter la prothèse 1 au regard des ouvertures des branchements des vaisseaux secondaires 12. Cela permet au chirurgien un gain de temps substantiel au cours de l'opération chirurgicale. Le godet 4 est maintenu par défaut dans une position pliée par des moyens de fixation 14 amovible. On entend par moyens de fixation un élément de fixation. Le godet 4 est apte à prendre successivement une position pliée et une position déployée dans laquelle il est destiné à coopérer avec une prothèse collatérale 10. En position pliée, le godet 4 forme un pli d'aisance 15. On entend par pli d'aisance 15 que la gaine 2 est rabattue sur elle-même pour former une double épaisseur. Le pli d'aisance 15 est maintenu par des moyens de fixation amovible 14. En position pliée, le godet 4 s'inscrit dans le pourtour de la gaine 2. Le godet 4 s'aligne dans le diamètre de la prothèse 1. Il suit le profil de l'armature 3 Le godet 4 ne forme pas de dépression dans la lumière 19 de la prothèse 1. Il n'y a pas de gêne possible du flux sanguin. La position pliée du godet 4 est maintenue lorsque le godet 4 ne coopère pas avec une prothèse collatérale 10. Le pli d'aisance 15 est aussi maintenu au cours de la mise en place de la prothèse. Dès qu'une prothèse collatérale 10 est mise en place, le pli d'aisance 15 est déplié et le godet 4 est en position déployée de sorte à recevoir la prothèse collatérale 10. La prothèse collatérale 10 s'applique sur la face externe de la prothèse 1 et plus particulièrement de la gaine 2 au niveau du godet 4.

Les moyens de fixation 14 sont amovibles pour permettre la libération du godet 4. Par exemple par inflation d'un ballon lors de la mise en place de la prothèse collatérale 10.

Les moyens de fixation amovibles 14 sont choisis notamment parmi des agrafes à détordre, des ancres dont les branches s'adossent, des noeuds dans un fil cassable, une colle polymère, un laçage par exemple autoserrant ou autodesserrant. A titre d'exemple, un fil de 8/0 voire 10/0, est satisfaisant. Préférentiellement, les moyens de fixation amovible 14 sont agencés sur la face externe de la gaine 2. De cette manière, lorsque le godet 4 se déploie, les moyens de fixation 14 sont retenus sur la face externe de la gaine 2 et ne risquent pas d'être emportés dans le flux sanguin.

Cette position pliée du godet 4 est particulièrement avantageuse lorsque la prothèse 1 comprend une pluralité de godets 4. Ainsi, les godets 4 non utilisés sont maintenus en position pliée et n'interfèrent pas dans la lumière 19 de la prothèse 1.

A titre d'exemple préféré, la prothèse 1 présente un diamètre déployé de 24 à 38 mm tandis que les prothèses collatérales 10 présentent des diamètres de 5 à 9 mm. Un godet 4 en position déployée représente de 5 à 20% du diamètre de ladite prothèse 1.

Le godet 4 est configuré pour coopérer avec une prothèse collatérale 10. En référence aux figures 1 et 2, des exemples indicatifs de godet 4 et de pli d'aisance 15 sont décrits ci-après.

Le godet 4 y présente une longueur 21 correspondant à la longueur de la gaine 2 entre les deux sommets proximaux 6a successifs entre lesquels le godet 4 est formé. La hauteur 16 du godet 4 correspond à la distance, parallèle à l'axe longitudinal 23 de la prothèse 1, entre le point le plus distal et le point le plus proximal du godet 4.

Selon un mode de réalisation, la hauteur 16 du godet 4 déployé correspond à la somme de la hauteur 17 du stent en zigzag et de la profondeur 22 du godet 4. La profondeur 22 du godet 4 correspond à la différence entre la longueur curviligne 21 du godet 4 préférentiellement au niveau de la bordure de la gaine 2 et la distance 20 entre deux sommets proximaux 6a successifs entre lesquels le godet 4 est formé. La profondeur 22 s'entend suivant une direction transversale à l'axe longitudinal 23. Par exemple, la profondeur 22 est choisie pour être au moins égale au demi-diamètre de la prothèse collatérale 10 qui va coopérer avec le godet 4. La profondeur 22 du godet correspond à la dépression formée dans la lumière 19 de la prothèse 1. La hauteur 16 du godet 4 est adaptée pour permettre la mise en parallèle de la prothèse collatérale 10 depuis le branchement avec le vaisseau secondaire 12. Le godet 4 s'étend avantageusement depuis le branchement avec le vaisseau secondaire 12 jusqu'à l'extrémité, préférentiellement proximale, de la gaine 2.

A titre d'exemple,

- hauteur 17 du stent en zigzag = distance CD = 16mm ;
- distance 20 inter-sommets proximaux = distance AB = distance A'B' = 15mm ;
- profondeur 22 du godet 4 = distance C'D' = 3 mm ;
- longueur 21 du godet 4 = distance A'C'+C'B' = 18 mm ;
- hauteur 16 du godet 4 = distance CD + D'C' = 19 mm.

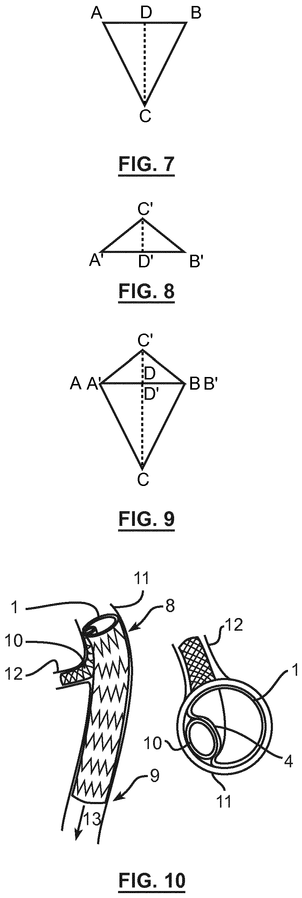

Pour mettre en oeuvre un godet 4 selon l'invention on peut notamment schématiser le godet 4 comme illustré aux figures 7 à 9. Le godet est schématisé par un triangle ABC, fig. 7, correspondant à un triangle du stent en Z où est formé le godet 4, et un triangle A'B'C', fig.8. Les bases AB et A'B' des deux triangles étant accolées pour former le godet 4, Fig. 9.

La distance 20 inter-sommets proximaux correspond aux distances AB et A'B'= 15mm. Les hauteurs des deux triangles étant DC et D'C'. D'C', correspondant à la profondeur 22 du godet 4, est choisi égale à 3 mm correspondant sensiblement à un demi-diamètre de la prothèse collatérale 10. On peut calculer sur la base d'un triangle rectangle A'D'C' :

$$A'C'^2 = A'D'^2 + D'C'^2$$

$$A'C'^2 = 7.5^2 + 3'^2$$

$$A'C' = \sqrt{7,5} + \sqrt{3}$$

$$A'C' = 8,87$$

La distance A'C' peut être arrondie à 9mm soit une distance A'C'+C'B' de 18mm correspondant à la longueur 21 du godet 4.

En position pliée, avantageusement position par défaut, le godet 4 est sous forme d'un pli d'aisance 15. La hauteur 18 du pli d'aisance 15 correspond à la longueur de la partie de la gaine 2 qui est repliée sur elle-même pour former le pli. Avantageusement, la hauteur 18 du pli est égale à la profondeur 22 du godet 4. En effet, la profondeur 22 du godet 4 est la surlongueur de la gaine 2 par rapport à la distance 21 entre les deux sommets proximaux 6a. C'est cette surlongueur qu'il faut replier pour que la gaine 2 se positionne suivant le pourtour de la prothèse 1 et ne forme pas de dépression dans la lumière 19. Lorsque le godet 4 est en position pliée, la longueur 21 de la gaine 2 entre deux sommets proximaux successifs 6a est égale à la distance 20 entre les deux dits sommets 6a. La gaine 2 ne contraint pas la distance entre les deux sommets.

Selon une possibilité illustrée sur l'ensemble des figures, l'extrémité proximale de la gaine 2 coïncide avec l'extrémité proximale de l'armature 3 et forme donc l'extrémité proximale de la prothèse 1.

Selon une autre possibilité, l'armature 3 se prolonge au-delà de l'extrémité proximale de la gaine 2. L'extrémité proximale de la gaine 2 est agencée au niveau de points de contact sur l'armature 3. Pour la description, les points de contact correspondent aux sommets proximaux 6a de l'armature 3.

La prothèse 1 est configurée pour prendre une position comprimée qui est utilisée pour sa mise en place et une position déployée dans laquelle elle est soit en position dans un vaisseau sanguin en appui sur les parois internes du vaisseau, soit déployée avant utilisation.

Selon un aspect séparable de l'invention, la prothèse endovasculaire pour montage en cheminée comprend une armature 3 tubulaire associée à une gaine 2 tubulaire, la prothèse étant configurée pour prendre au moins une position comprimée et une position déployée. La gaine 2 et l'armature 3 sont en contact à l'extrémité, préférentiellement proximale, de la gaine 2 au niveau de deux points de contact et de deux points d'appui sur l'armature 3. En position déployée, la distance 21 entre les deux points de contact de ladite gaine 2 est supérieure à la distance 20 entre les deux points d'appui de l'armature 3. Cette différence de longueur génère une dépression dans la gaine 2 destinée à coopérer avec une prothèse collatérale 10. Préférentiellement, cette différence de longueur est maintenue sur une partie de la hauteur de la prothèse 1. Selon une possibilité, cette différence de longueur est croissante sur la hauteur de la prothèse 1 en direction de l'extrémité proximale 8 de la prothèse 1. Il y a formation d'un godet 4 en direction de l'extrémité proximale 8 de la prothèse 1. La prothèse 1 est introduite dans l'artère à traiter de manière connue en comprimant la prothèse 1. Une fois la prothèse 1 placée dans la zone de l'artère 11 à traiter, la prothèse 1 est déployée, soit par ballon soit automatiquement dès le retrait de la contrainte de compression. La circulation sanguine est rétablie au travers de la lumière 19. Le positionnement de la prothèse 1 est uniquement vérifié de manière à ce que la zone à traiter, c'est-à-dire par exemple l'anévrisme, soit couverte et que la prothèse 1 s'étende suffisamment de part est d'autre pour maintenir une étanchéité satisfaisante. Il n'est pas nécessaire avec la prothèse 1 selon l'invention de contrôler le positionnement radiale de la prothèse 1 au regard des ouvertures de branches des vaisseaux secondaires 12. Une prothèse collatérale 10 est introduite dans un vaisseau secondaire 12 débouchant au niveau de la prothèse 1. La prothèse collatérale 10 est introduite de manière connue sous forme comprimée. La prothèse collatérale 10 est positionnée partiellement dans le vaisseau secondaire 12 et partiellement en parallèle de la prothèse 1 dans l'artère 11. Préférentiellement, la prothèse collatérale 10 est disposée à l'extérieur de la prothèse 1. Le positionnement longitudinal de la prothèse collatérale 10 est tel que ladite prothèse 10 débouche dans l'artère 11 au moins au niveau de l'extrémité proximale 8 de la prothèse 1 voire après ladite extrémité 8. La prothèse collatérale 10 est avantageusement positionnée en parallèle de la prothèse 1 au niveau d'un godet 4, pouvant être en position pliée sous forme d'un pli d'aisance 15. La prothèse collatérale 10 est déployée par un ballon et/ou automatiquement dès le retrait de la contrainte de compression. La circulation sanguine est rétablie au travers de la prothèse collatérale 10. Le déploiement de la prothèse collatérale 10 entraine le déploiement du godet 4 avec la rupture des moyens de fixation 14 s'ils sont présents. Les moyens de fixation 14 sont configurés pour être désolidarisés du pli d'aisance 15 par l'expansion de la prothèse collatérale 10.

Dans le cas d'un fil, celui-ci est suffisamment fragile pour être cassé par la force d'expansion de la prothèse collatérale. A titre d'exemple, des fils de 8/0 ou 10/0 ont montré une rupture satisfaisante. Dans le cas d'agrafe ou d'ancre, la résistance à la torsion est choisie pour être plus faible que la force d'expansion de la prothèse collatérale 10.

Pour la colle polymère, la résistance à l'arrachage est également sélectionnée pour être inférieure à la force d'expansion de la prothèse collatérale 10.

Les autres plis d'aisance 15 maintenus par défaut ou par des moyens de fixation 14 et qui ne sont pas utilisés pour le passage d'une prothèse collatérale 10 restent en position pliée. L'expansion de la prothèse principale 1 n'entraîne pas l'inactivation des moyens de fixation 14.

Le pli d'aisance 15 ne contribue pas à réduire le périmètre de la prothèse 1. Le pli d'aisance 15 ne s'oppose pas à l'expansion de la prothèse 1, plus particulièrement à l'expansion de l'armature 3.

En partie proximale notamment, le contact entre la prothèse 1 et les parois de l'artère 11 est maximal, la prothèse 1 grâce au godet 4 ne se déforme pas. La prothèse 1 maintient une forme sensiblement circulaire. La prothèse 1 entoure la prothèse collatérale 10 au niveau du godet 4 pour optimiser les contacts avec les parois de l'artère 11. La figure 14 illustre cette disposition qui est avantageuse par rapport aux prothèses classiques de l'état de la technique illustrées en figure 13 où l'on voit la prothèse 1 déformée par la prothèse collatérale empêchant un contact suffisant entre la prothèse 1 et les parois de l'artère 11 générant des endofuites entre les deux prothèses 1,10.

## REFERENCES

1. Prothèse
2. Gaine
3. Armature
4. Godet
5. Fil métallique
6a. Sommets proximaux
6b. Sommets distaux
7. Traverse/segment droit
8. Extrémité proximale
9. Extrémité distale
10. Prothèse collatérale
11. Artère
12. Vaisseaux secondaires
13. Circulation sanguine
14. Moyens de fixation
15. Pli d'aisance
16. Hauteur du godet
17. Hauteur du Z
18. Hauteur du pli d'aisance
19. Lumière
20. Distance inter-sommets proximaux
21. Longueur du godet
22. Profondeur du godet
23. Axe longitudinal de la prothèse

## Revendications

1. Prothèse (1) endovasculaire adaptée au montage en cheminée comprenant une gaine (2) et une armature (3) tubulaires définissant une lumière (19); la prothèse (1) comprend à une extrémité de la gaine (2) au moins un godet (4) formé en direction de ladite extrémité de sorte à former une dépression dans la lumière (19), dépression destinée à coopérer avec une prothèse (10) endovasculaire collatérales, **caractérisée par le fait que** le godet (4) est maintenu par défaut dans une position pliée sous la forme d'un pli d'aisance (15) maintenu par des moyens de fixation (14) amovibles.

**2.** Prothèse (1) selon la revendication précédente dans laquelle le godet (4) est formé par la gaine (2).

**3.** Prothèse (1) selon la revendication précédente dans laquelle au niveau du godet (4), l'armature (3) est configurée pour laisser le godet (4) libre de déformation.

**4.** Prothèse (1) selon l'une quelconque des revendications précédentes dans laquelle la gaine (2) recouvre au moins partiellement l'armature (3).

**5.** Prothèse (1) selon l'une quelconque des revendications précédentes dans laquelle l'armature (3) comprend au niveau de l'extrémité proximale de la gaine (2) un fil métallique périphérique disposé en « z » et comprenant des sommets proximaux (6a) dirigés vers l'extrémité proximale, le au moins un godet (4) étant formé entre deux sommets proximaux successifs (6a).

**6.** Prothèse (1) selon l'une quelconque des revendications précédentes dans laquelle les moyens de fixation (14) sont agencés sur la face externe de la gaine (2).

**7.** Prothèse (1) selon l'une quelconque des revendications précédentes dans laquelle le pli d'aisance (15) formé comprend une composante principale orientée suivant l'axe longitudinal (23) de la prothèse (1).

**8.** Prothèse (1) selon l'une quelconque des revendications précédentes dans laquelle le pli d'aisance (15) aligne le godet (4) à la périphérie de l'armature (3).

**9.** Prothèse (1) selon l'une quelconque des revendications précédentes comprenant une pluralité de godets (4) sur la gaine (2).

**10.** Prothèse (1) selon la revendication précédente combinée avec la revendication 5 dans laquelle des godets (4) sont formés entre des sommets proximaux (6a) de l'armature (3) en « z ».

**11.** Système de prothèses endovasculaires adapté au montage en cheminée comprenant une prothèse principale (1) et une prothèse collatérale (10) destinées à être assemblées en parallèle dans un vaisseau sanguin humain ou animal **caractérisé par le fait que** la prothèse principale (1) est une prothèse selon l'une quelconque des revendications précédentes et qu'elle comprend à une extrémité au moins un godet (4) formé en direction de ladite extrémité de sorte à former une dépression recevant la prothèse collatérale (10) sur une portion de la surface extérieure de la prothèse principale (1).

**12.** Système selon la revendication précédente dans lequel le godet (4) a une profondeur (22) maximale égale au diamètre de la prothèse collatérale, préférentiellement la moitié du diamètre.

**Patentansprüche**

**1.** Für eine Kaminmontage geeignetes Implantat-Stent (1), bestehend aus einer röhrenförmigen Umhüllung (2) und einem röhrenförmigen Stützwerk (3), welche eine Öffnung (19) abgrenzen. Der Stent (1) weist an einem Ende der Umhüllung (2) mindestens eine Ausrundung (4) auf, die in Richtung des besagten Endes so geformt ist, dass eine Einsenkung in der Öffnung (19) besteht, wobei die Einsenkung dazu bestimmt ist, mit einem kollateralen Implantat-Stent (10) zusammen zu wirken, **dadurch gekennzeichnet dass** die Ausrundung (4) standardmäßig in einer gefalteten Position in Form einer Bewegungsfalte (15) gehalten wird, die durch abnehmbare Befestigungsmittel (14) fixiert wird.

**2.** Stent (1) nach dem vorhergehenden Anspruch, in dem die Ausrundung (4) durch die Umhüllung (2) geformt wird.

**3.** Stent (1) nach dem vorhergehenden Anspruch, in dem das Stützwerk (3) in Höhe der Ausrundung (4) so gestaltet ist, dass sich die Ausrundung (4) beliebig verformen kann.

**4.** Stent (4) nach einem der vorhergehenden Ansprüche, in welchem die Umhüllung (2) mindestens teilweise das Stützwerk (3) bedeckt.

**5.** Stent (1) nach einem der vorhergehenden Ansprüche, in welchem das Stützwerk (3) am proximalen Ende der Umhüllung (2) einen peripheren Metalldraht umfasst, der z-förmig angeordnet ist und proximale Spitzen (6a) aufweist, die zum proximalen Ende hinzeigen, wobei mindestens eine Ausrundung (4) zwischen zwei aufeinanderfolgenden proximalen Spitzen (6a) geformt ist.

**6.** Stent (1) nach einem der vorhergehenden Ansprüche, in welchem die Befestigungsmittel (14) an der Außenseite der Umhüllung (2) angeordnet sind.

**7.** Stent (1) nach einem der vorhergehenden Ansprüche, in welchem die geformte Bewegungsfalte (15) eine Hauptkomponente umfasst, die der Längsachse (23) des Stents (1) folgend ausgerichtet ist.

**8.** Stent (1) nach einem der vorhergehenden Ansprüche, in welchem die Bewegungsfalte (15) die Ausrundung (4) auf den Rand des Rahmens (3) ausrichtet.

9. Stent (1) nach einem der vorhergehenden Ansprüche, welcher eine Vielzahl von Ausrundungen (4) auf der Umhüllung (2) aufweist.

10. Stent (1) nach dem vorhergehenden Anspruch in Kombination mit dem Anspruch 5, in dem die Ausrundungen (4) zwischen den proximalen Spitzen (6a) des Stützwerks (3) z-förmig gebildet werden.

11. Für eine Kaminmontage geeignetes Implantat-Stent-System, welches eine Hauptprothese (1) und eine kollaterale Prothese (10) umfasst, die dazu bestimmt sind, in einem menschlichen oder tierischen Blutgefäß parallel montiert zu werden, **dadurch gekennzeichnet, dass** die Hauptprothese (1) eine Prothese nach einem der vorhergehenden Ansprüche ist und dass sie an einem Ende mindestens eine Ausrundung (4) umfasst, die in Richtung des besagten Endes so geformt ist, dass sie eine Einsenkung bildet, die die kollaterale Prothese (10) in einem Abschnitt der Außenoberfläche der Hauptprothese aufnimmt (1).

12. System nach dem vorhergehenden Anspruch, in dem die Ausrundung (4) eine maximale Tiefe (22) hat, die gleich dem Durchmesser der kollateralen Prothese ist, vorzugsweise die Hälfte des Durchmessers.

**Claims**

1. An endovascular prosthesis (1) suitable for chimney-grafting, comprising a tubular sheath (2) and framework-stent (3) defining an lumen (19);
the prosthesis (1) comprises, at one end of the sheath (2), at least one godet (4) formed toward said end so as to form a depression in the lumen (19), a depression able to mate with a collateral stent (10), **characterized in that** the godet (4) is held by default in a folded position in the form of a pleat (15) held by removable fastening means (14).

2. A prosthesis (1) according to the preceding claim, wherein the godet (4) is formed by the sheath (2).

3. A prosthesis (1) according to the preceding claim, wherein, at the godet (4), the framework (3) is so configured as to keep the godet (4) free of any deformation.

4. A prosthesis (1) according to any one of the preceding claims, wherein the sheath (2) at least partially covers the framework (3).

5. A prosthesis (1) according to any one of the preceding claims, wherein the framework (3) comprises, at the proximal end of the sheath (2), a peripheral metallic wire positioned in a "z"-shape and comprising proximal apexes (6a) oriented towards the proximal end, with the at least one godet (4) being formed between two successive proximal apexes (6a).

6. A prosthesis (1) according to any one of the preceding claims, wherein the fastening means (14) are arranged on the outer face of the sheath (2).

7. A prosthesis (1) according to any one of the preceding claims, wherein the pleat (15) formed comprises a main component oriented along the longitudinal axis (23) of the prosthesis (1).

8. A prosthesis (1) according to any one of the preceding claims, wherein the pleat (15) aligns the godet (4) at the periphery of the framework (3).

9. A prosthesis (1) according to any one of the preceding claims, comprising a plurality of godets (4) on the sheath (2).

10. A prosthesis (1) according to the preceding claim, combined with claim 5, wherein godets (4) are formed between proximal apexes (6a) of the "z"-shaped framework (3).

11. An endovascular prosthesis system suitable for chimney-grafting, comprising a main prosthesis (1) and a collateral prosthesis (10) intended to be assembled in parallel in a human or animal blood vessel,
**characterized in that** the main prosthesis (1) is a prosthesis according to any one of the preceding claims, and **in that** it comprises, at one end, at least one godet (4) formed toward said end, so as to form a depression receiving the collateral prosthesis (10) on a portion of the outer surface of the main prosthesis (1).

12. A system according to the preceding claim, wherein the godet (4) has a maximum depth (22) equal to the diameter of the collateral prosthesis, preferably half the diameter.

FIG. 1

FIG. 2

FIG. 3

FIG. 4

FIG. 5

FIG. 6

A    D    B

C

**FIG. 7**

C'

A'  D'  B'

**FIG. 8**

C'

A A'   D   B B'
D'

C

**FIG. 9**

11
1
8
10
12
12
9
13
1
10
4
11

**FIG. 10**

**FIG. 11**

**FIG. 12**

FIG. 13

FIG. 14

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20130103134 A1